# EUROPEAN PATENT SPECIFICATION

(11) **EP 0 233 607 B1**
(45) Date of publication and mention of the grant of the patent: **13.03.1991**
(21) Application number: 87102046.7
(22) Date of filing: 13.02.1987
(51) Int. Cl.: C07C 239/20, C07D 205/08

(54) **[(2,2,2-Trichloroethoxy) methoxy]amine**
[(2,2,2-Trichloroethoxy)methoxy]amin
[(2,2,2-Trichloroéthoxy)méthoxy]amine

(30) Priority: 18.02.1986 US 830050
(43) Date of publication of application: 26.08.1987
(73) Proprietor: E.R. Squibb & Sons, Inc., Princeton, N.J. 08540-4000 (US)
(72) Inventor: Denzel, Theodor, Regensburg 84 (DE); Schierling, Peter, Regensburg (DE)
(74) Representative: Perani, Aurelio

(56) References cited:
- EP-A- 0 170 280
- THE JOURNAL OF ORGANIC CHEMISTRY, vol. 50, no. 2, 25th January 1985, pages 169-175, The American Chemical Society; A.G.M.BARRETT et al.: "Acyl and sulfonyl isocyanates in beta-lactam synthesis"

## Description

This invention is directed to the novel chemical compound [(2,2,2-trichloroethoxy)methoxy]-amine, which is useful for the preparation of 3-acylamino-1-hydroxy-2-azetidinones which are in turn useful for the preparation of antibacterial agents.

The novel chemical compound [(2,2,2-trichloroethoxy)methoxy]amine has the formula 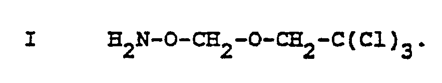
It is a protected oxyamine which is particularly useful in synthetic applications which require the presence of an oxyamine group that is protected against hydrogenolysis and protected against both acidic and basic conditions.

[(2,2,2-Trichloroethoxy)methoxy]amine can be used, for example, in the preparation of hydroxamates having the formula 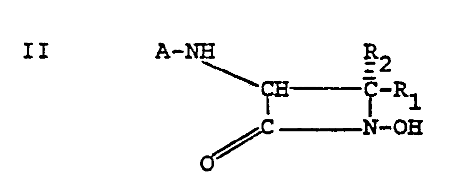
wherein A is an amino protecting group and R₁ and R₂ are the same or different and each is hydrogen or alkyl. These hydroxamates of formula II are useful intermediates for the preparation of antibacterial agents; e.g., 3-acylamino-2-oxoazetidin-1-yloxy acetic acids, which are disclosed in United States patent application Serial No. 515,727 filed July 21, 1983 and United Kingdom Patent Specification 2,125,794, published March 14, 1984, and 3-acylamino-2-oxo-1-azetidinyl sulfates, which are disclosed in United States patents 4,337,197 and 4,533,660, issued June 29, 1982 and August 6, 1985 respectively. These references also describe procedures for preparing the hydroxamates of formula II.

Hydroxamates of formula II can be prepared from amino acids having the formula 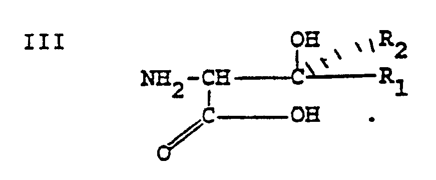
The amino group is first protected with a classical protecting group (e.g., t-butoxycarbonyl, benzyloxycarbonyl, o-nitrophenylsulfenyl, etc; the protecting group is represented by the symbol "A"), yielding a compound of the formula 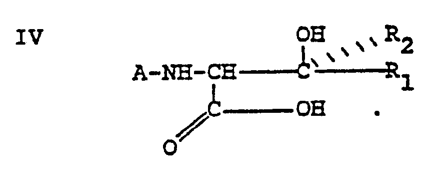
The carboxyl group of a protected amino acid of formula IV is then reacted with a protected oxyamine. The oxyamine of this invention, [(2,2,2,-trichloroethoxy)methoxy]amine, is particularly useful for this reaction, and yields the corresponding compound having the formula 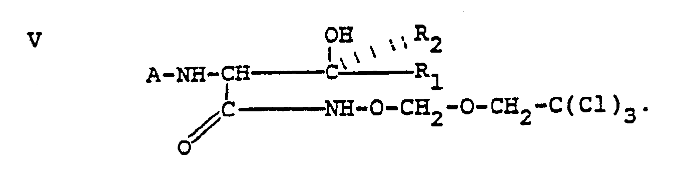
The reaction proceeds most readily in the presence of a coupling agent such as 1-ethyl-3-(3-dimethyl-aminopropyl)carbodiimide or dicyclohexylcarbodiimide.

Conversion of a compound of formula V to a protected hydroxamate having the formula 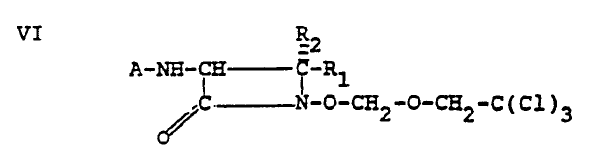
can be accomplished by
1) converting the hydroxyl group of a compound of formula V to a leaving group (e.g., by reaction with methane-sulfonyl chloride) followed by treatment of the fully protected compound with a base;
2) treatment of a compound of formula V with triphenylphosphine and diethyl-azodicarboxylate; or
3) sulfonating a compound of formula V with pyridine (or substituted pyridine) sulfur trioxide complex and cyclizing the resulting compound by treatment with a base.

Conversion of a compound of formula VI to a hydroxamate of formula II can be accomplished by treating the compound of formula VI with zinc dust.

[(2,2,2-Trichloroethoxy)methoxy]amine can be prepared by reacting (2,2,2-trichloroethoxy)methyl chloride with N-hydroxyphthalimide to yield N-[(2,2,2-trichloroethoxy)methoxy]phthalimide. The reaction is preferably carried out in an organic solvent (e.g., dimethylformamide) in the presence of an inorganic base (e.g., potassium carbonate).

Conversion of N-[(2,2,2-trichloroethoxy)-methoxy]phthalimide to [(2,2,2-trichloroethoxy)-methoxy]amine can be accomplished by treating the compound with hydrazine hydrate.

The starting compound (2,2,2-trichloroethoxy)methyl chloride is known; see Acta. Chem. Scand., 14: 777-779 (1960).

The following examples further illustrate this invention.

### Example 1

### [(2,2,2-Trichloroethoxy)methoxy]amine

### A) N-[(2,2,2-Trichloroethoxy)methoxy]phthalimide

N-Hydroxyphthalimide (163.1 g) was dissolved in 1.6 l of dimethylformamide with stirring at 20°C. (2,2,2-Trichloroethoxy)methyl chloride (197.9 g) was added followed by 138.2 g of finely ground potassium carbonate. The mixture was stirred at 20-30°C for 5 hours, then poured into 3 l of ice cold water with vigorous agitation. After 2 hours, the crystalline solid was filtered, washed with about 6 l of water until the cake was colorless and dried at 40°C in vacuo to give 268 g of the title compound, melting point 122°C.

### B) [(2,2,2-Trichloroethoxy)methoxy]amine

N-[(2,2,2-Trichloroethoxy)methoxy]phthalimide (268 g) was dissolved in 1.6 l of dichloromethane. Hydrazine hydrate (79.8 g) was added and stirring was continued for 5 hours at 20°C. The precipitated hydrazide was filtered, washed with dichloromethane and discharged. The solvent was removed in vacuo, and the oily residue was distilled to give 152.8 g of [(2,2,2-trichloroethoxy)methoxy]amine, boiling point 47-49°C at 0.5 mm of Hg.

### Example 2

### Use of [(2,2,2-trichloroethoxy)methoxy]amine to prepare [3S]-3-[[(phenylmethoxy)carbonyl]amino]-1-hydroxy-4,4-dimethyl-2-azetidinone

### A) N²-[(Phenylmethoxy)carbonyl]-N-[(2,2,2-trichlororthoxy)methoxy]-L-β-hydroxyvalinamide

N-[(Phenylmethoxy)carbonyl]-L-β-hydroxyvaline (178 g) was dissolved in 1.7 l of ethyl acetate. After cooling to 5°C, 128.2 g of [(2,2,2-trichloroethoxy)methoxy]amine, 148.6 g. of dicyclohexylcarbodiimide and 5 g of 1-hydroxybenzotriazole were added with stirring. Cooling was removed arid stirring was continued for 12 hours. The precipitated dicyclohexylurea was filtered off, washed with 200 ml of ethyl acetate and discharged. The solvent was evaporated in vacuo. Toluene (400 ml) was added and the solvent was again removed in vacuo to a final residue of about 500 g. Some seeds were added, followed by 80 ml of n-hexane. The mixture was stirred with cooling to 5°C for 2 hours until crystallization began. Additional n-hexane (100 ml) was added. After 15 minutes, the precipitated crystals were filtered and dried at 40°C in vacuo yielding 265.3 g of the title compound.

### B) [3S]-3-[(Phenylmethoxy)carbonyl]amino]-1-[(2,2,2-trichloroethoxy)methoxy]-4,4-dimethyl-2-azetidinone

Pyridine (71 g) was dissolved in 400 ml of dichloroethane and cooled to - 20°C. Chlorosulfonic acid (34.8 g) was added with stirring at a rate that the temperature did not exceed - 5°C. After complete addition, the cooling was stopped and stirring was continued for 15 minutes. N²-[(Phenylmethoxy)carbonyl]-N-[(2,2,2-trichloroethoxy)methoxy]-L-β-hydroxyvalinamide (44.4 g) was added and the mixture was heated to 60°C for 2 hours. The warm mixture was then slowly added to a solution of 100 g of potassium bicarbonate in 400 ml of water and heated to reflux for 3 hours, maintaining the pH of the mixture at 8.5 by the addition of either potassium carbonate or hydrochloric acid as necessary. After cooling to 20°C, the phases were separated. The aqueous phase was once extracted with 100 ml of dichloroethane. The organic layers were combined and the pH was adjusted to 2.8 by the addition of diluted phosphoric acid. The phases were separated and the aqueous phase was again extracted with 100 ml of dichloroethane. The combined organic layers were washed with 100 ml of water and concentrated in vacuo. Butyl acetate (120 ml) was added. Solvent (80 ml) was distilled off in vacuo. The residue was cooled to 5°C with stirring for 30 minutes. N-Hexane (30 ml) was added and stirring was continued for an additional 30 minutes. The precipitated crystals were collected, washed with butyl acetate/n-hexane (1: 3) and dried at 30°C to give 23 g of the title compound.

### C) [3S]-3-[(Phenylmethoxy)carbonyl]amino-1-hydroxy-4,4-dimethyl-2-azetidinone

[3S]-3-[[(Phenylmethoxy)carbonyl]amino]-1-[(2,2,2-trichloroethoxy)methoxy]-4,4-dimethyl-2-azetidinone (20 g) was dissolved in 50 ml of acetic acid and 30 ml of tetrahydrofuran. Zinc dust (20 g) was added and the temperature was allowed to rise to 30-35°C. After 2 hours, the reaction was complete and 400 ml of butyl acetate was added. The unreacted zinc was removed by filtration. The solution was washed with water and brine and 300 ml of the solvent was removed in vacuo. N-Hexane (10 ml) was added, followed by some seeds. After crystallization, the mixture was cooled to 5°C, 100 ml of n-hexane was added and stirring was continued for 1 additional hour. The precipitated crystals were filtered, washed with n-hexane and dried in vacuo to yield 11 g of the title compound.

## Claims

1. [(2,2,2-Trichloroethoxy)methoxy]amine

2. A process for preparing a compound having the formula 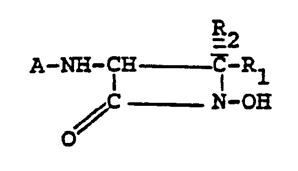 which comprises reacting a compound having the formula 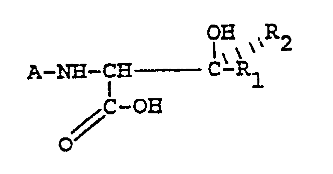 with [(2,2,2-trichloroethoxy)methoxy]amine and cyclizing the resultant compound having the formula 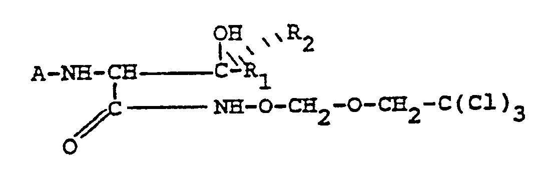 to obtain a compound having the formula 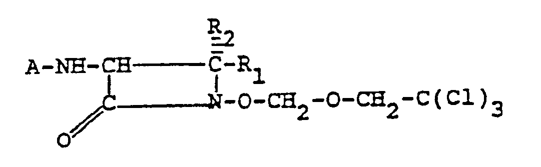 and converting that compound to the desired hydroxamate; wherein A is an amino protecting group and R₁ and R₂ are the same or different and each is hydrogen or alkyl.

3. A process in accordance with claim 2 wherein cyclization is accomplished by converting the hydroxyl group of the compound having the formula 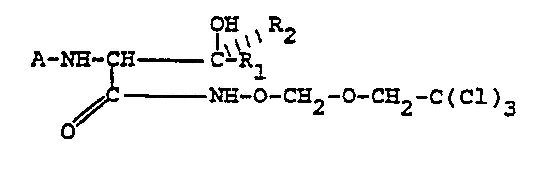 to a leaving group followed by treatment with a base.

4. A process in accordance with claim 2 wherein cyclization is accomplished by treatment of the compound having the formula 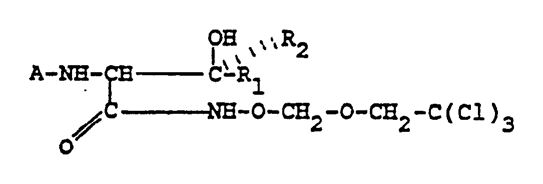 with triphenylphosphine and diethylazodicarboxylate.

5. A process in accordance with claim 2 wherein cyclization is accomplished by sulfonating a compound having the formula 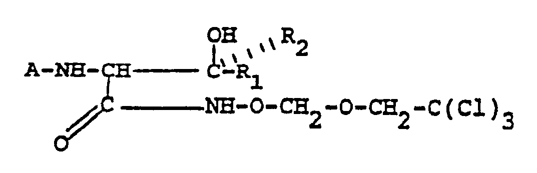 with pyridine, or substituted pyridine, sulfur trioxide complex followed by treatment with a base.

6. A process in accordance with claim 2 wherein the compound having the formula 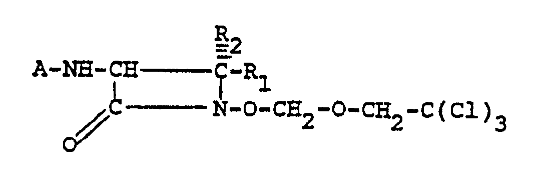 is converted to the desired hydroxamate by treatment with zinc dust.

7. A process in accordance with claim 2 wherein R₁ and R₂ are each methyl.

## Patentansprüche

1. [(2,2,2-Trichlorethoxy)-methoxy]-amin.

2. Verfahren zur Herstellung einer Verbindung der Formel 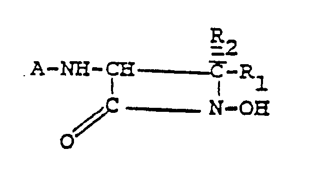 umfassend die Umsetzung einer Verbindung der Formel 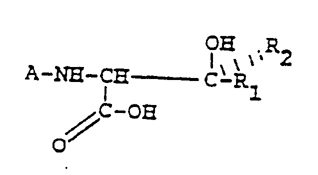 mit [(2,2,2-Trichlorethoxy)-methoxy]-amin und die Cyclisierung der erhaltenen Verbindung der Formel 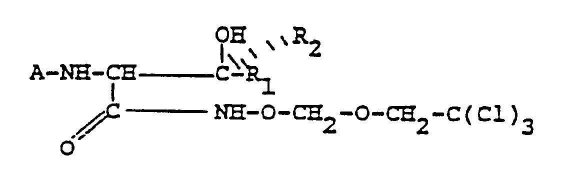 zu einer Verbindung der Formel 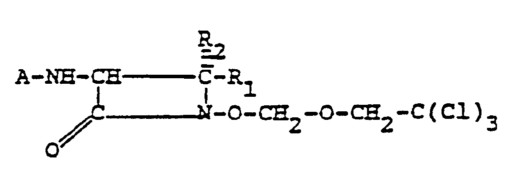 und die Umwandlung dieser Verbindung in das gewünschte Hydroxamat, wobei A eine Aminschutzgruppe bedeutet und R₁ und R₂ gleich oder verschieden sind und jeweils ein Wasserstoffatom oder einen Alkylrest bedeuten.

3. Verfahren nach Anspruch 2, wobei die Cyclisierung durch Umwandlung der Hydroxylgruppe der Verbindung der Formel 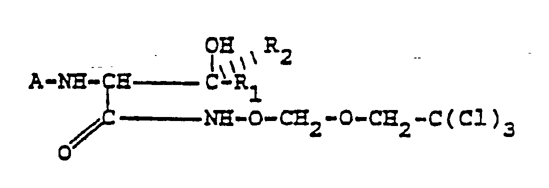 in eine Abgangsgruppe, gefolgt von einer Behandlung mit einer Base, erreicht wird.

4. Verfahren nach Anspruch 2, wobei die Cyclisierung durch Behandlung der Verbindung der Formel 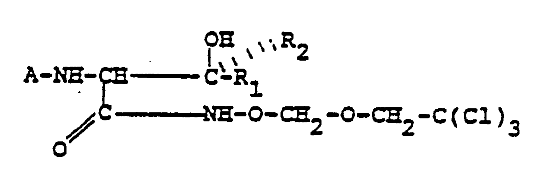 mit Triphenylphosphin und Diethylazodicarboxylat erreicht wird.

5. Verfahren nach Anspruch 2, wobei die Cyclisierung durch Sulfonierung einer Verbindung der Formel 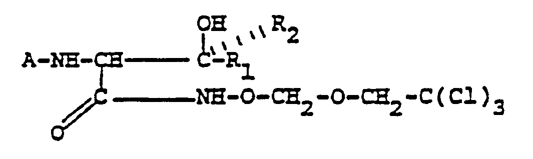 mit Pyridin oder einem substituierten Pyridin, Schwefeltrioxid-Komplex, gefolgt von einer Behandlung mit einer Base, erreicht wird.

6. Verfahren nach Anspruch 2, wobei die Verbindung der Formel 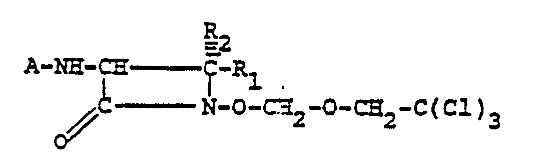 durch Behandlung mit Zinkstaub in das gewünschte Hydroxymat umgewandelt wird.

7. Verfahren nach Anspruch 2, wobei R₁ und R₂ jeweils eine Methylgruppe bedeuten.

## Revendications

1. [(2,2,2,-Trichloréthoxy)méthoxy] amine.

2. Procédé de préparation d'un composé de formule 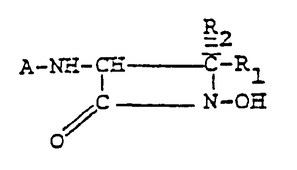 qui consiste à faire réagir un composé de formule 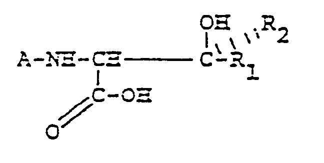 avec la [(2,2,2-trichloréthoxy)méthoxy] amine et à cycliser le composé résultant, de formule 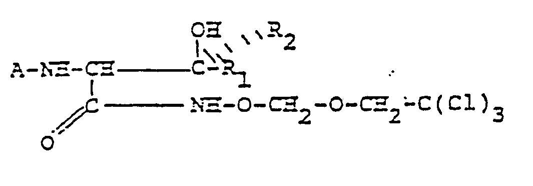 pour obtenir un composé de formule 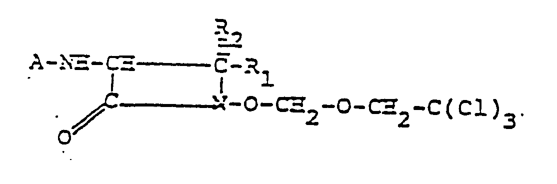 et à transformer ce composé en l'hydroxamate voulu; A étant un groupe protecteur de la fonction amine et R₁ et R₂ étant identiques ou différents et étant chacun l'hydrogène ou un radical alkyle.

3. Procédé selon la revendication 2, dans lequel on réalise la cyclisation en transformant le groupe hydroxyle du composé de formule 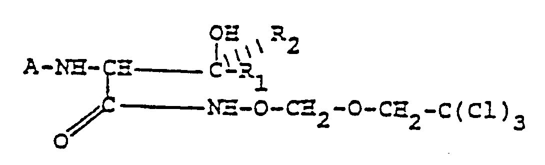 en un groupe labile, puis en traitant par une base.

4. Procédé selon la revendication 2, dans lequel on réalise la cyclisation en traitant le composé de formule 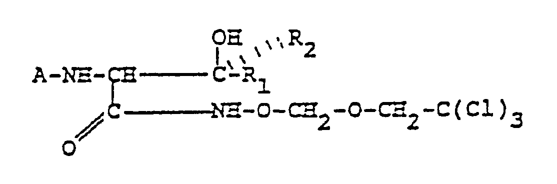 par la triphénylphosphine et le diéthylazodicarboxylate.

5. Procédé selon la revendication 2, dans lequel on réalise la cyclisation en sulfonant un composé de formule 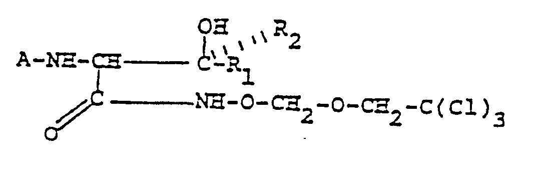 à l'aide d'un complexe de pyridine, ou de pyridine substituée, et de trioxyde de soufre, puis en traitant par une base.

6. Procédé selon la revendication 2, dans lequel on transforme le composé de formule 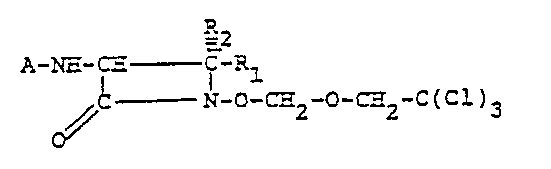 en l'hydroxamate voulu en le traitant par de la poudre de zinc.

7. Procédé selon la revendication 2, dans lequel R₁ et R₂ sont tous deux des radicaux méthyle.
